# EUROPEAN PATENT SPECIFICATION

(11) **EP 4 100 728 B1**
(45) Date of publication and mention of the grant of the patent: **02.04.2025**
(21) Application number: 21710342.3
(22) Date of filing: 03.02.2021
(51) Int. Cl.: G01N 27/00, B82Y 30/00, B82Y 15/00, G01N 27/26, G01N 27/327, G01N 27/48, G01N 33/487, G01N 33/49, G01N 33/493

(54) **METHOD FOR FUNCTIONALISING A CELLULOSE SUPPORT WITH METAL NANOPARTICLES AND ELECTROANALYTICAL SENSOR COMPRISING THE FUNCTIONALISED CELLULOSE SUPPORT**
VERFAHREN ZUM FUNKTIONALISIEREN VON ZELLULOSETRÄGERN MIT METAL NANOPARTIKELN UND ELEKTROANALYTISCHER SENSOR MIT FUNKTIONALISIERTEM ZELLULOSETRÄGER
PROCEDE POUR LA FUNCTIONALISATION DE SUPPORTS DE CELLULOSE AVEC NANOPARTICULES MÉTALLIQUES ET CAPTEUR ÉLECTROANALYTIQUE AVEC SUPPORT DE CELLULOSE FONCTIONNALISÉ

(30) Priority: 03.02.2020 IT 202000002017
(43) Date of publication of application: 14.12.2022
(73) Proprietor: Cardiovascular Lab S.p.A., 20123 Milano (IT)
(72) Inventor: MAZZARACCHIO, Vincenzo, 20123 Milano (IT); BAGHERI, Neda, 20123 Milano (IT); CINTI, Stefano, 20123 Milano (IT); MOSCONE, Danila, 20123 Milano (IT); ARDUINI, Fabiana, 20123 Milano (IT)
(74) Representative: Studio Torta S.p.A.
(86) International application number: PCT/IB2021/050869
(87) International publication number: WO 2021/156763

(56) References cited:
- WO-A1-2010/073260
- CN-A- 104 122 393
- CN-A- 105 675 597
- CN-A- 109 061 190

## Description

### CROSS-REFERENCE TO RELATED APPLICATIONS

This patent application claims priority from Italian patent application no. 102020000002017 filed on 03/02/2020.

### TECHNICAL FIELD

The present invention relates to a method for functionalising a cellulose support with metal nanoparticles, to a method for producing an electroanalytical sensor comprising said functionalised cellulose support, to a method for producing the electroanalytical sensor, and to a method for detecting at least a marker in a biological fluid involving the use of said sensor.

### BACKGROUND

The possibility of detecting particular markers in biological fluids, such as blood, sweat, saliva or urine, in a simple and economical way, without the need for laboratories or specialised personnel, is becoming increasingly important in the field of analytical chemistry.

In this sense, electrochemical sensors made on cellulose supports, in particular paper, have been developed and represent a cost-effective and ecological solution at the same time.

Cinti S. et al. (2018), Talanta 179: 186-192 describes an electrochemical sensor on office paper for detecting chloride ions in serum and sweat.

Cinti S. et al. (2017), Sensors and Actuators B 253:1199-1206 describes an electrochemical sensor on office paper for detecting zinc ions in serum and sweat.

Cinti S. et al. (2018), Talanta 187: 59-64 describes an electrochemical sensor on filter paper for detecting glucose in blood sample. In this article Prussian Blue nanoparticles are used to catalyse the reduction of hydrogen peroxide, which is produced by oxidation of glucose by glucose oxidase. For Prussian Blue nanoparticles to form, it is necessary to use solutions that contain not only the different precursors of the nanoparticles but also a reducing agent.

The aforesaid sensors still have limits in terms of efficiency and the use of cellulose, in particular of paper, has problems, in particular in detecting analytes such as metals, as the non-specific adsorption of the metals on the porous structure of the support could prevent the accumulation of the analyte at the working electrode. This problem is even more important if metals are to be detected at very low concentrations (for example free copper which has physiological concentrations below 20 ppb).

CN 105675597 and WO 2010/073260 describe the functionalisation of cellulose substrates with platinum and silver nanoparticles respectively obtained using the respective precursors and reducing species.

It is clear how developing functional and efficient supports for the production of innovative electrochemical sensors, which solve the problems currently encountered, in particular with respect to some specific markers such as metals, today not otherwise measurable outside specialised laboratories, represents a key component for the future and for the sustainability and applicability of the latest generation electroanalytical sensors.

### DISCLOSURE OF INVENTION

It is therefore an object of the present invention to provide a method for functionalising, in particular by conferring electrocatalytic properties and capacity of concentrating analytes, a cellulose support with metal nanoparticles, which allows solving the aforementioned problems in a simple and efficient way and whose production on industrial scale is entirely feasible through processes and machinery already used for the production of printed electrochemical sensors.

This object is achieved by the present invention as it relates to a method as defined in claim 1.

A further object of the present invention is to provide a cellulose support functionalised by means of metal nanoparticles formed in situ as defined in claim 2.

It is also an object of the present invention to provide a method for producing an electroanalytical sensor as defined in claim 4.

Finally, objects of the present invention are to provide an electroanalytical sensor as defined in claim 6 and a method for detecting a marker in a biological fluid as defined in claim 9.

### BRIEF DESCRIPTION OF THE FIGURES

Figure 1 represents a schematic view of a sensor according to the invention with reference to a preferred embodiment.
Figure 2 represents the steps of the method according to the invention with reference to the production and use of the sensor according to the preferred embodiment of Figure 1.
Figure 3 shows graphs and images of the sensors relative to the concentration and volume optimisation experiments of the HAuCl₄ solution used to functionalise a sensor according to the preferred embodiment of Figure 1.
Figure 4 shows graphs relative to the comparison of electrochemical performances of a sensor with paper functionalised with AuNP according to the preferred embodiment of Figure 1 and a similar sensor with non-functionalised paper.
Figure 5 shows graphs of the optimisation of the deposition potential, the deposition time, the scan rate and the conditioning potential for the sensor according to the preferred embodiment of Figure 1.
Figure 6 shows a graph comparing the performances of a sensor with AuNP-functionalised paper versus a non-functionalised sensor using samples containing increasing concentrations of copper ions. The relative calibration curve is shown in the box.
Figure 7 shows the graph relative to a test of the sensor according to the preferred embodiment of Figure 1 using two different concentrations of copper ions (200 ppb and 300 ppb).
Figure 8 shows a graph showing the performances of a sensor according to the preferred embodiment of Figure 1 in which the AuNPs have been functionalised in situ compared to an analogous sensor in which the AuNPs are pre-constituted and deposited on paper.
Figure 9 shows graphs showing the different accuracy in the determination of H₂O₂ (hydrogen peroxide), a classic product of multiple enzymatic reactions, detectable by an immunoenzymatic sensor, using reduction potentials from - 0.3V to -0.5V in case of functionalisation of paper with metal nanoparticles compared with non-functionalised paper support.
Figure 10 shows scanning electron microscopy (SEM) images showing how the metal nanoparticles formed in situ are distributed. Figures 10A and 10B are relative to functionalised paper while Figure 10C is relative to non-functionalised paper.
Figure 11 shows a graph with the distribution of the dimensions of the metal nanoparticles formed in situ detected by dynamic light scattering (DLS) analysis.
Figure 12 shows a graph comparing the performances of an immunoenzymatic sensor on paper functionalised (decorated) with AuNP and an immunoenzymatic sensor on non-functionalised paper.

### DETAILED DESCRIPTION OF THE INVENTION

The method for functionalising a cellulose support with metal nanoparticles according to the present invention comprises the steps of: depositing on the cellulose support a single aqueous solution of the metal precursor, in the form of acid or salt, in a concentration from 1 to 6 mM; and placing the cellulose support at a temperature from 65°C to 80°C for a time from 10 to 40 minutes.

For the functionalisation of the metal nanoparticles, it is not necessary to add a reducing compound. Instead, the solution consists only in the metal precursor.

Preferably the aqueous solution of the metal precursor is a solution of tetrachloroauric acid (HAuCl₄), preferably at a concentration of about 2.5 mM. Alternatively, use can be made of solutions of silver salts, preferably silver nitrate (AgNO₃) and silver acetate (AgCH₃COO); bismuth salts, preferably bismuth nitrate (Bi(NO₃)₃) ; cobalt salts, preferably cobalt acetate (Co(CH₃COO)₂) and cobalt sulphate (CoSO₄); selenious acid (H₂SO₃); copper salts, preferably copper sulphate (CuSO₄) and copper acetate (Cu(CH₃COO)₂; hexachloroplatinic acid (H₂PtCl₆) and platinum salts, preferably potassium tetrachloroplatinate (K₂PtCl₄) ; palladium salts, preferably palladium dichloride (PdCl₂) and sodium tetrachloropalladate (Na₂PdCl₄); nickel salts, preferably nickel chloride (NiCl₂) and nickel nitrate (Ni(NO₃)₂), to obtain respectively silver, bismuth, cobalt, selenium, copper, platinum, palladium and nickel nanoparticles.

It is possible to consider the use of particular precautions, such as a controlled atmosphere to avoid oxidation of the nanoparticles.

The cellulose support is preferably paper, more preferably filter paper.

The deposit surface is lower than 0.6 cm². Preferably the deposit surface is from 0.2 cm² to 0.6 cm².

The cellulose support is preferably placed at a temperature of about 70°C for about 30 minutes.

In virtue of to the aforesaid method, a cellulose support functionalised with metal nanoparticles is obtained. The particular treatment and the precursor solution according to the aforesaid method allow to obtain particular structural characteristics of the nanoparticles inserted in the cellulose structure, which give the functionalised support either an electrocatalytic function or a concentration function of the analytes.

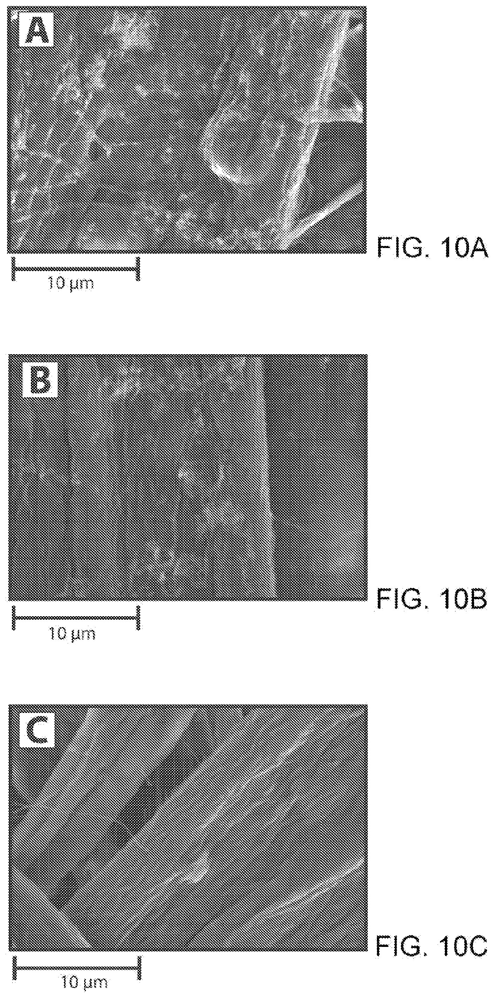

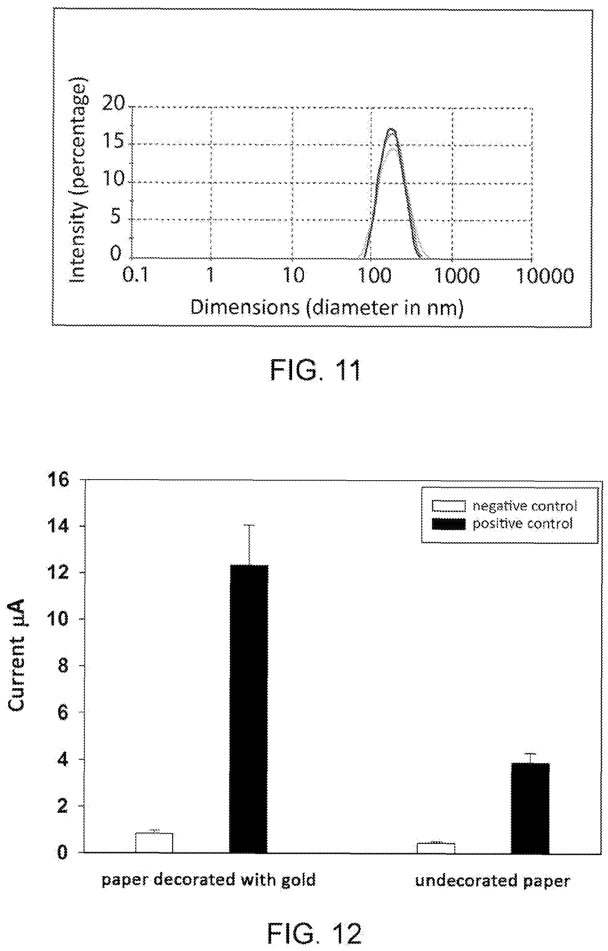

As shown below in Example 11 (Figure 10), the metal nanoparticles form aggregates on the cellulose support, defined as hot spots, which are very different from the aggregates formed by nanoparticles previously formed and then deposited on the support at a later time as well as also with respect to the aggregates formed by nanoparticles formed using a solution of metal precursor and subsequently a solution with a reducing compound. As further shown in Example 12 (Figure 11), the metal nanoparticles have average dimensions equal to 196.2 ± 20.7 nm with a polydispersity index equal to 0.13.

The larger dimensions of the nanoparticles obtained according to the invention allow a better conduction when used on an electrochemical sensor. The result is an increased sensitivity of the sensor, which can then be used for the detection of metals in biological liquids such as serum.

The functionalised cellulose support as described above can in fact be used in the production of single electroanalytical sensors or inserted in multiple sensor platforms for the detection of one or more markers (analytes) in a biological fluid.

The method for producing an electroanalytical sensor for detecting a marker in a biological fluid according to the present invention comprises the following steps.

A cellulose support is provided. Preferably, said support is formed of paper, preferably filter or office paper, more preferably filter paper, in particular filter paper with a weight in the range between 60-85 g/m², more preferably 67 g/m², Cordenons, Italy, or whatman cellulose filter paper can be used.

A hydrophilic working area is then delimited on the cellulose support, which is naturally hydrophilic, by depositing a hydrophobic material. The hydrophobic material can be wax and be printed on the substrate.

The hydrophilic working area thus delimited is the deposit area of the metal precursor; preferably it is lower than 0.6 cm², more preferably it corresponds to a range from 0.2 cm² to 0.6 cm². A single aqueous solution of the metal precursor is then deposited on the hydrophilic working area of the cellulose support, in the form of acid or salt of a metal in a concentration from 1 to 6 mM. As already described above, the solution, containing only the precursor of the metal is preferably a solution of tetrachloroauric acid (HAuCl₄) and the concentration of the acidic aqueous solution of the metal is preferably of about 2.5 mM.

The cellulose support is then placed at a temperature from 65°C to 80°C, preferably 70°C, for a time from 10 to 40 minutes, preferably about 30 minutes, so that metal nanoparticles are formed on the cellulose support.

On the hydrophilic working area of the cellulose support, at least one working electrode, one reference electrode and one counter-electrode are then printed by screen-printing, by depositing conductive inks in succession.

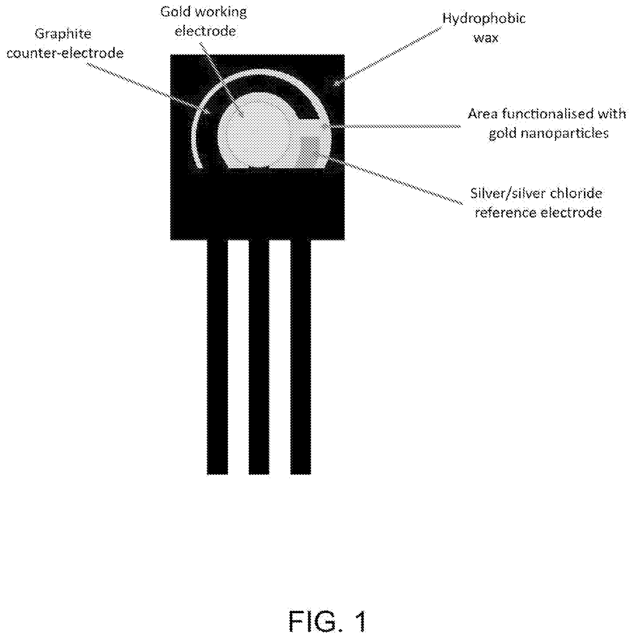

Preferably, a gold-based ink is used for the working electrode, a silver/silver chloride-based ink is used for the reference electrode, and a graphite-based ink is used for the counter-electrode. It is possible to coat the working electrode with materials with a filter or barrier function, to reduce the risk of biofouling and further improve the performance of the sensor. With reference to Figure 1, the working electrode is preferably printed in a central position with respect to the reference electrode and the counter-electrode. The working electrode can have an area of about 7 mm².

The electroanalytical sensor for the detection of a marker in a biological fluid according to the invention therefore comprises a cellulose support functionalised with metal nanoparticles formed in situ, on which a hydrophobic area delimits a hydrophilic working area, said hydrophilic working area comprising at least one working electrode, one reference electrode and one counter-electrode printed by screen-printing.

The in situ synthesised nanoparticles are metallic. The metal is preferably Au, Ag, Bi, Co, Se, Cu, Pt, Pd, Ni. More preferably, the metal nanoparticles are made of Au.

The deposit surface is lower than 0.6 cm². Preferably the deposit surface is from 0.2 cm² to 0.6 cm².

The biological fluid is preferably blood, serum, urine or sweat or saliva or tear fluid or synovial fluid, more preferably blood.

The marker is preferably a metal selected from the group consisting of Cu, Fe, Zn, Pb, Hg, Cd, and As. More preferably the metal is Cu.

In a preferred embodiment, the working electrode is gold, the reference electrode is silver/silver chloride and the counter-electrode is graphite.

In a preferred embodiment, the sensor is immunoenzymatic and exploits the antigen-antibody interaction to bind the molecule of interest and then an enzymatic reaction to produce the signal to be measured. In particular, the sensor can detect serum proteins, transferrin, ferritin, haemoglobin, vitamins, antibodies, cytokines. The marker in case of immunoenzymatic sensor is preferably H₂O₂, ascorbic acid, nitrophenol, hydroquinone.

The method for detecting at least one marker in a biological fluid according to the present invention comprises the steps of providing a sensor as previously described; adding to said sensor a quantity of biological fluid from 1 to 80 µl, preferably from 20 to 40 µl, more preferably 30 µl, where said biological fluid has optionally undergone a pre-treatment by dilution or concentration; preferably the deposit of the solution takes place on a single side of the paper support, therefore either on the front side (the side on which the electrodes are printed) or on the rear side; more preferably it takes place on the front side; applying a potential difference between the sensor electrodes, in one or more passages, concentrating the marker at the working electrode; and detecting a current signal by means of a potentiostat which is proportional to the amount of marker in the biological fluid.

Preferably, a time-varying potential is applied which allows the oxidation of the analyte and a consequent current signal. More preferably, a potential fixed over time (DEPOSITION POTENTIAL) is applied at a determined value to reduce and concentrate the analyte on the working electrode and then a second time-varying potential is applied to allow the oxidation of the analyte and a consequent current signal.

In particular, the potentiostat detects a current which is proportional to the concentration of the marker in the biological fluid.

The electroanalytical sensor on a functionalised cellulose support described above can be used in a microfluidic platform coupled to other sensors printed on cellulose or other materials.

### Example 1 - production of a sensor for the detection of copper

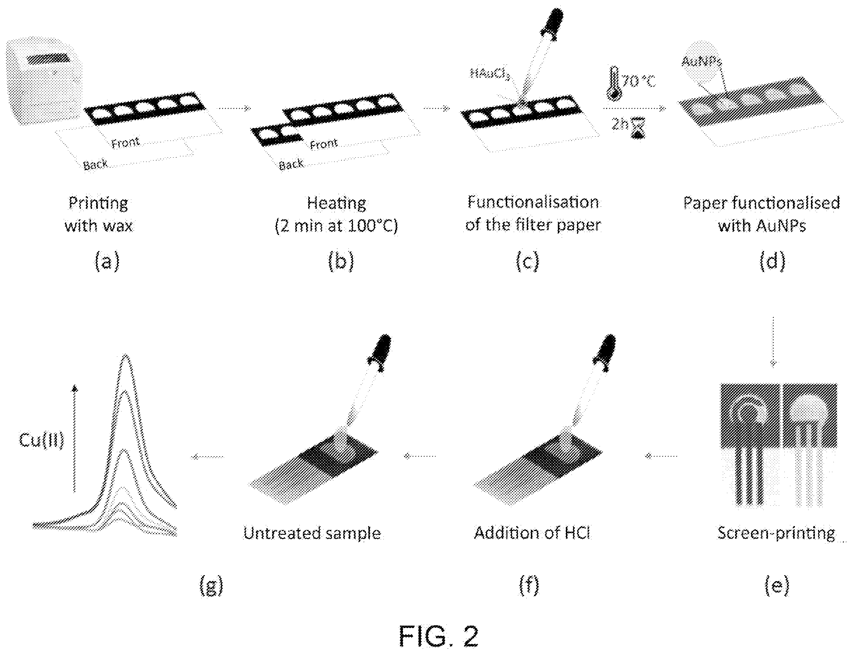

With reference to Figure 2, a hydrophobic wax pattern was printed on a sheet of filter paper (67 g/m², Cordenons, Italy) by means of a solid ink printer (Xerox ColorQube 8580), necessary to delimit the area on which the gold nanoparticles will be synthesised and the electrodes printed. In order to create the hydrophobic barrier, the filter paper is placed in a stove at 100°C for 2 minutes to allow the wax to melt in the paper.

4 µL of a HAuCl₄ solution at a concentration of 2.5 mM, prepared in ultrapure distilled water, by placing the support at 70°C for 30 min, in order to synthesise the nanoparticles, were deposited on the area delimited by the hydrophobic barrier.

Subsequently, on this functionalised surface, the electrodes were printed by successive depositions of conductive inks, using the screen-printing technique. For this purpose, a gold-based ink (BQ331, DuPont, France) was used for the working electrode, a silver/silver chloride-based ink (Electrodag 477 SS, Acheson, Italy) for the reference and a graphite-based ink (Electrodag 421, Acheson, Italy) for the counter-electrode. Each ink requires a passage in the oven of respectively 20 minutes for the silver chloride and graphite ink, and of 40 minutes for the gold ink, at 70°C.

The gold working electrode (diameter = 7 mm²) is printed in a central position with respect to the reference and working electrode.

5 µl of HCl 0.4 M are deposited on the hydrophilic area of the electrode (semi-circular area) and allowed to evaporate at room temperature in order to have a ready-to-use sensor without the need for the operator to add other reagents apart from the sample to analyse.

The measurement of the copper ions is carried out by depositing 30 µL of the solution under analysis (sweat samples) directly on the functionalised area, in direct contact with the printed electrodes and carrying out an analysis by means of linear sweep anodic stripping voltammetry (LS-ASV) in the potential range from -0.5 to 0.6 V.

### Example 2

The volume and concentration of the HAuCl₄, solution, and the synthesis time of the nanoparticles were optimised in order to reach the method and the sensor of Example 1. The optimisation steps were monitored by cyclic voltammetry with a scan rate of 0.05 V/s in the presence of 5 mM iron/ferrocyanide prepared in 0.1 M KCl.

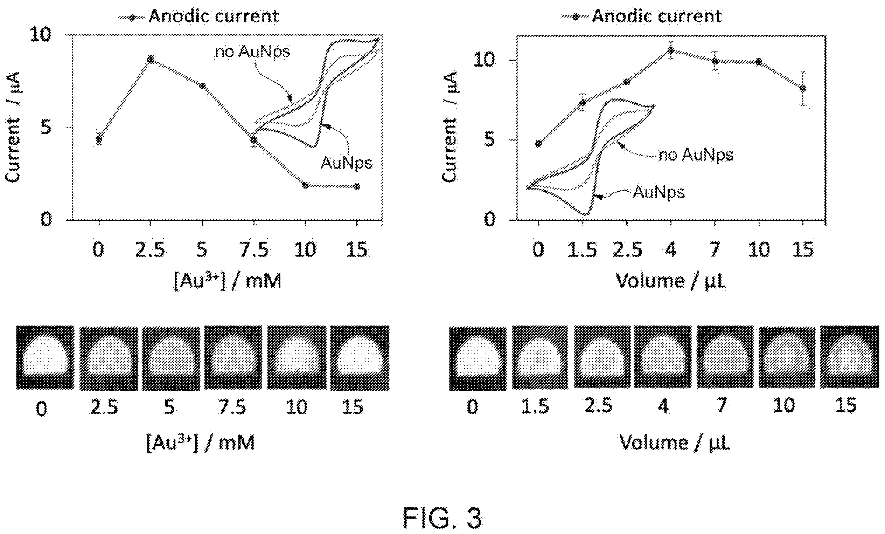

The results are represented in Figure 3. Figure 3A shows the concentration and Figure 3B shows the volume of the HAuCl₄ solution that is deposited on the paper to synthesise the gold nanoparticles (AuNP). The study on the concentration was carried out in the range between 0 and 15 mM, while the study on the volume was assessed between 0 and 15 µl. The points shown are the intensity of the anodic peak obtained in the presence of 5 mM iron/ferrocyanide by cyclic voltammetry at a scan rate of 0.05 V/s. All experiments were repeated in triplicate. As regards the optimisation of the HAuCl₄ concentration, it is evident how the colour of the synthesised nanoparticles changes when the concentration is greater than 2.5 mM. The colour of the AuNPs depends on the dimensions of the nanoparticles. The dark aspect represents the formation of aggregates which are not stable. This behaviour is also highlighted through the observation of the anodic current intensities: lower currents are recorded when the HAuCl₄ concentration is greater than 2.5 mM, which has been chosen as the optimal concentration.

After optimising the concentration, the assessment of the effect of the volume was carried out. The effect of the volume is mainly a consequence of the diffusion of the aqueous solution in the test area. If the volume is too low, the drop does not cover the whole area, if the volume is too high there is an accumulation of the dissolved species on the periphery. The optimal volume was identified to be 4 µl. As evident from Figure 3B the result is confirmed in terms of current intensity and colour/homogeneity of the AuNPs. Moreover, it is evident that a volume greater than or equal to 10 µl produces an accumulation effect of the species dissolved in the periphery.

The last optimisation step was on the heating time and formation of the nanoparticles (data not shown). The ideal compromise was found at 30 minutes at a temperature of 70°C.

### Example 3

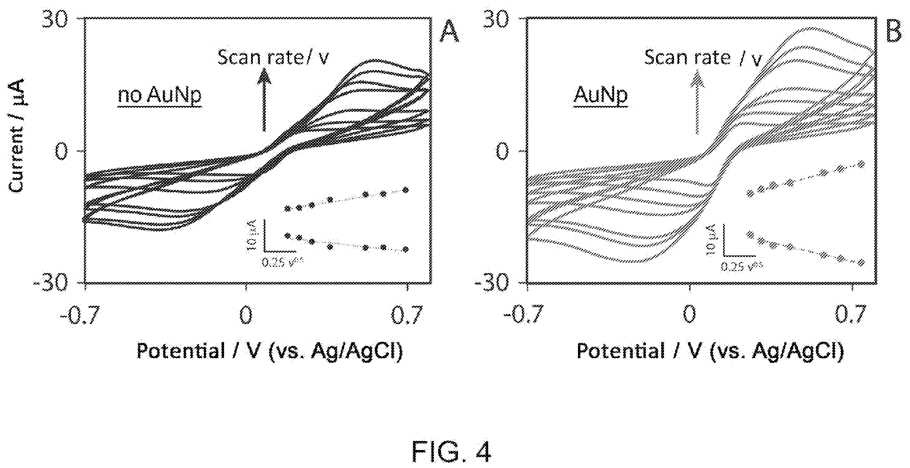

Following the optimisations described in Example 2, the electrochemical performances of the sensor with functionalised paper versus the sensor with non-functionalised paper was compared. The electrochemical efficiency was assessed in the presence of a 5 mM iron/ferrocyanide mixture by cyclic voltammetry experiments varying the scan rate from 0.02 to 1 V/s using unmodified paper (Figure 4A) and modified with AuNP (Figure 4B).

Both sensors follow the Randles-Sevcik equation (iₚ = (2.69 × 10⁵)n^{1.5}ACD^{0.5}v^{0.5}) and the linear correlation between current and square root of the scan rate confirms the mass transfer diffusion of the analyte. Furthermore, this behaviour is consistent with the absence of trapping phenomena of the analyte on the working electrode.

As evident from the figures (Figures 4A and 4B), the presence of AuNP in the filter paper produces an increase in the current intensity (about twice) and an increase in the kinetics of transfer of the electrons to the solution/electrode interface (the peak-to-peak separation (ΔE) at 20 mV/s decreased from 210 to 140 mV when AuNP nanoparticles are present).

### Example 4

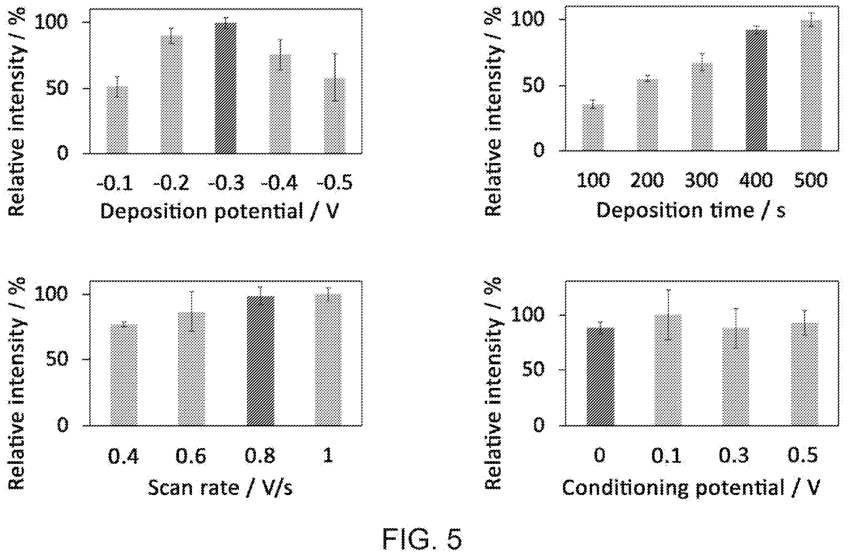

For the development of the method and sensor described in Example 1, the electrochemical parameters for the detection of copper in relation to the anodic redissolution voltammetry were optimised. In particular, as shown in Figure 5, the deposition potential, the deposition time, the scan rate and the conditioning potential were assessed. The initial conditions for the first parameter that have been optimised (deposition potential) are: t dep= 200 s; scan rate = 0.8 V/s; E cond= 0 V. All the experiments were conducted in the presence of 200 ppb Cu (II) (prepared in a 0.1 M HCl solution).

### Example 5

Since the detection of copper ions is typically performed in a highly acidic environment, a comparison was made between a procedure in which the AuNP-functionalised sensor was first impregnated with HCl and then dried and then a solution of copper ions was applied, and a procedure in which the same sensor was not impregnated, but a solution of copper ions containing HCl was applied. In both cases the same sensitivity was obtained, demonstrating that it is possible to apply the HCl solution to the sensor beforehand and then provide the sensor ready for use without the operator having to add other reagents apart from the sample to be analysed.

### Example 6

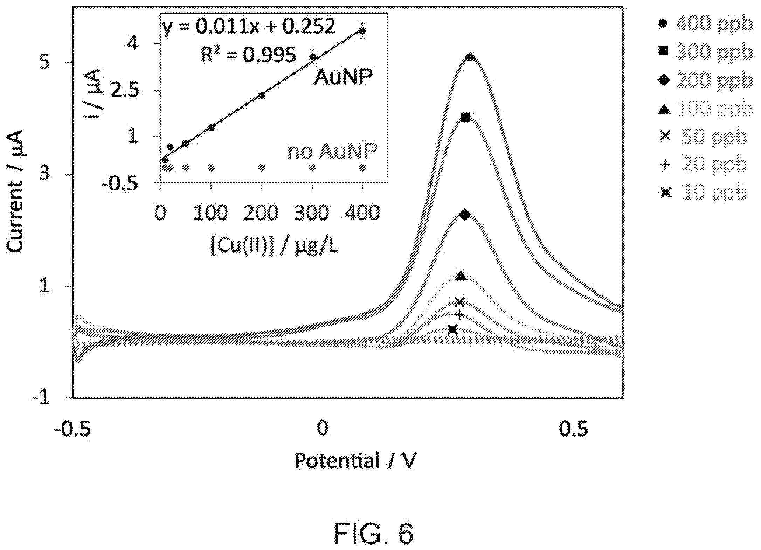

As shown in Figure 6, a calibration curve was obtained by analysing distilled water with known concentrations of copper ions. The results obtained by means of a sensor constituted with AuNP-functionalised paper were compared with the results obtained using a non-functionalised sensor. In particular, copper ion concentrations from 10 to 400 µg/L were tested.

The results with functionalised paper are represented in Figure 6 with solid lines, while the results with non-functionalised paper are represented by a dashed line. All the measurements were performed by impregnating the working area with 20 µl of copper ion solution in double distilled water using the parameters used in Example 4. The inner box of Figure 6 shows the comparison between calibration curves obtained through non-functionalised and functionalised paper.

The use of functionalised paper highlighted a linear correlation between current intensity (deriving from the subtraction of the signal obtained in the absence of analyte, y) and the concentration of copper ions (expressed in µg/L, x) through the following equation : y = 0.011x + 0.252 with R² = 0.995. The detection limit (3σ_{B}/method sensitivity) calculated as the ratio between 3 times the standard deviation of the signal obtained in the absence of analyte (σ_{B}) and the method sensitivity, defined as the slope of the linear section of the calibration curve resulted in 3 µg /L, and the limit of quantification was equal to 10 ppb. The response was linear in the range between 10 and 400 µg/L. As evident from the inner box of Figure 6 it is evident that it is the presence of AuNP that allows the detection of copper ions.

### Example 7

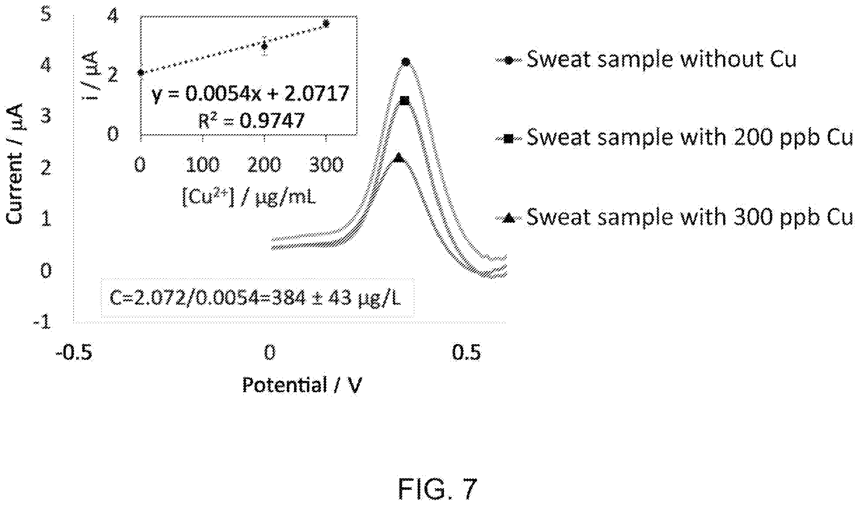

The performances of the sensor with functionalised paper were tested for the detection of copper ions in sweat samples. 20 µl of sweat obtained from volunteers who had done physical activity with different concentrations of copper (200 and 300 µg/L) were deposited on the working area of the sensor. As shown in Figure 7, by adding known concentrations of copper ions (200 and 300 µg/L) and using the standard addition method, it was possible to quantify the presence of copper ions in sweat samples.

### Example 8

The accuracy of the method was successfully assessed either by performing two-level recovery studies or by validating the results, comparing them with those obtained with the use of atomic absorption spectroscopy (AAS), as shown in the following Table 1.

**Table 1**

| | **Recoveries** | | | | | |
|---|---|---|---|---|---|---|
| Sweat sample #1 | Added concentration (µg/L) | | Recovery (%) | | RSD (%) | |
| | 100 | | 98 | | 4 | |
| | 250 | | 82 | | 9 | |

| | **Validation** | | | | | |
|---|---|---|---|---|---|---|
| Sweat sample #2 | Detected copper ions (µg/L) | | F-Test | | Student's t-test | |
| | AuNP sensor | AAS | Sp. value | Critical value | Sp. value | Critical value |
| | 384 ± 43 | 391 ± 10 | 18.49 | 19.00 | 0.137 | 2.776 |

The levels of copper ions that were detected in untreated sweat are in accordance with the physiological values (25-2100µg/L), and the comparison with the reference method for the detection of copper ions (AAS) provided a good correlation within the degree of experimental uncertainty.

### Example 9

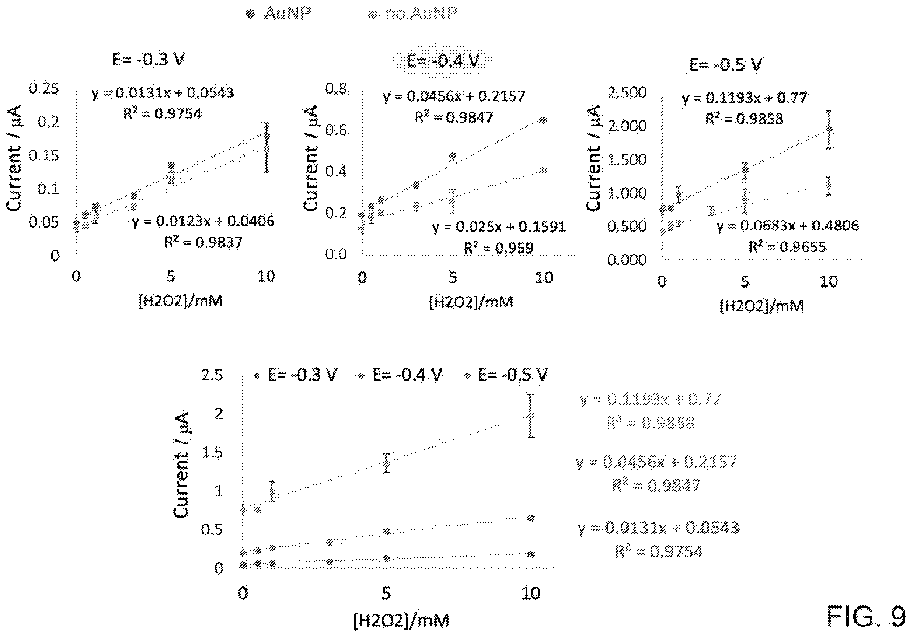

In this example (Figure 9) it was successfully demonstrated that the functionalisation of paper with metal nanoparticles increases the sensitivity in the detection of the products of immunoenzymatic type electrochemical sensors. Specifically, an increase in the capability of accurately detecting the immunoenzymatic sensor marker H₂O₂ (hydrogen peroxide) using an AuNP-functionalised paper and by applying a reduction potential preferably between -0.3V and -0.5V was demonstrated.

### Example 10

In this example it was shown that the functionalisation of the filter paper with pre-constituted AuNPs or with metal precursors from which nanoparticles are generated in situ differs substantially.

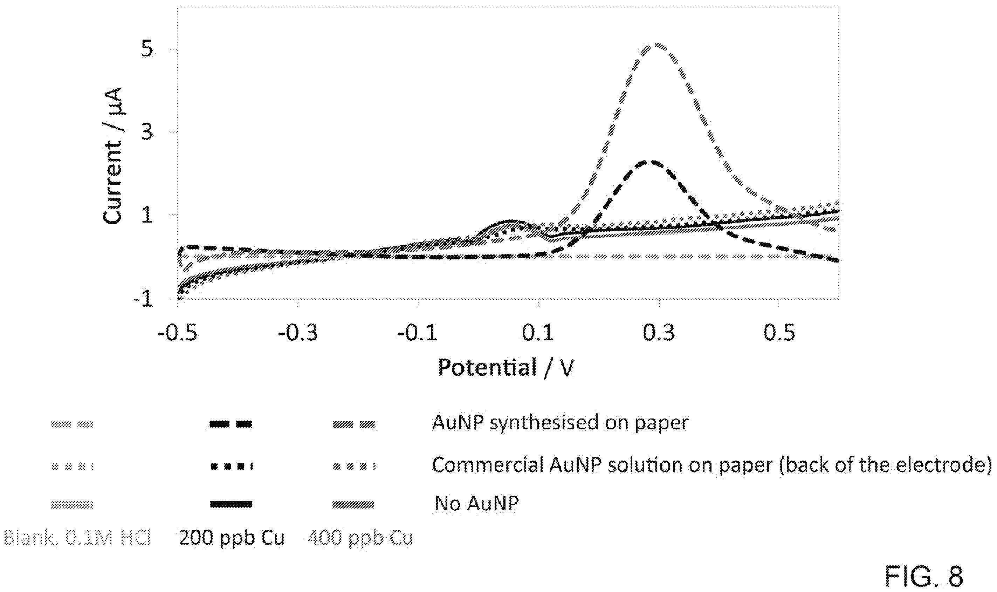

Specifically, the functionalisation of paper with pre-constituted AuNPs, directly added on paper, does not give a good functional result, because the paper does not acquire an electro-catalyst function. This is evident from tests carried out using the proposed sensor for the detection of copper ions. As shown in Figure 8, the sensor for the detection of copper when printed on paper directly functionalised with pre-constituted AuNP does not work (the current peak is flat). Conversely, it works much better when the paper is functionalised starting from the metal precursor.

A possible explanation is that the in situ synthesis of the nanoparticles allows the metal precursor to be inserted in the cellulose network and the nanoparticles created in situ from the functional point of view are different from those deposited as such on paper.

### Example 11

A more detailed structural analysis was performed using SEM (scanning electron microscopy) in order to study the morphological characteristics of AuNP-functionalised cellulose substrates. Figures 10A-B and 10C show SEM images of AuNP-functionalised paper and non-functionalised paper, respectively. In detail, the microscopy showed that the AuNPs were dispersed and adsorbed on the paper surface forming hot-spots (Figures 10A and 10B). No AuNPs were detected on the non-functionalised paper (Figure 10C).

### Example 12

A dynamic light scattering (DLS) analysis was performed in order to understand the dimensions distribution of the nanoparticles. DLS measurements revealed a monodisperse AuNP suspension (Figure 11) with an average diameter of 196.2 ± 20.7 nm and a satisfactory polydispersity index (PDI) of 0.13.

### Example 13

A test was performed simulating a generic immunosensor (with activity comparable to any other more specific immunosensor) and alkaline phosphatase used as a label for assessing if paper functionalised with gold nanoparticles in situ modifies the signal detection through immunosensors. The response of the sensor printed on paper functionalised with gold nanoparticles created in situ was much better than that given by the sensor printed on the same undecorated paper. In particular, a dose of a generic antibody (20 µl) conjugated with the alkaline phosphatase enzyme (1µg/mL), prepared in phosphate saline buffer solution (PBS) was placed on the electrochemical sensor printed on paper. Then the enzyme reagent was added: 70 µL of 1-naphthyl phosphate (5 mg/mL, prepared in DEA-MgCl2-KCl buffer pH 9.6). After 2 minutes of enzymatic reaction, the electroactive product (1-naphthol) derived from the reaction was measured in differential pulse voltammetry (DPV) with the following parameters: Ebegin = -0.2 V; Eend = 0.4 V; Estep = 0.016 V; Epulse = 0.05 V; tpulse = 0.06 s; scan rate = 0.016 V/s. The measurement was repeated in triplicate, for each sensor (either decorated sensor or undecorated sensor). The results are indicated in Figure 12.

### Advantages

Significantly better sensor performances are obtained in virtue of the fact that the cellulose support, and not the inks for the electrode printing, is functionalised with metal nanoparticles in terms of either conductivity or concentration of the analyte to the electrode, in particular in the case in which the analyte to be measured is a metal. In fact, in the absence of such functionalisation, in the detection of analytes of biological fluids, the diffusion of the biological fluid sample inside the porous structure of the paper support generally prevents the analyte from accumulating at the working electrode. Vice versa, the functionalisation of the cellulose support with metal nanoparticles allows the detection of analytes, even at low concentrations (lower than 20 ppb), which cannot be highlighted with the sensor printed on non-functionalised cellulose. Furthermore, it is more advantageous to use a functionalised support rather than functionalising the electrodes: in particular, it is often difficult to mix an organic-based conductive ink with nanoparticles in aqueous solution. In the present method, instead, the metal nanoparticles are formed directly in the structure of the paper without the need to use additional reducing agents such as sodium borohydride, ascorbic acid or citrate. Also from the functional point of view, the metal nanoparticles synthesised in situ as per described optimised method have important advantages even regardless of the geometric shape and constituent material of the single electrodes: in fact, no equally satisfactory performances are obtained using pre-constituted metal nanoparticles.

Furthermore, the production of electroanalytical sensors, using the proposed method, does not involve the use of new technologies and therefore it can be easily implemented using processes and machinery already used for the industrial production of printed electrodes. Finally, cellulose supports functionalised with metal nanoparticles according to the method described, can also be used in microfluidic platforms for multiple sensors.

## Claims

1. A method for functionalising a cellulose support in situ with metal nanoparticles having electrocatalytic properties, the method comprising the steps of:
- depositing on a cellulose support a single aqueous solution of the metal precursor not including an additional reducing agent, in the form of acid or salt, in a concentration from 1 to 6 mM; and
- drying the cellulose support at a temperature from 65°C to 80°C for a time from 10 to 40 minutes.

2. A cellulose support functionalised with metal nanoparticles formed in situ obtained by the method according to claim 1.

3. Use of the cellulose support according to claim 2 in the production of immunoenzymatic electroanalytical sensors or electroanalytical sensors for the detection of at least one metal selected from the group consisting of Cu, Fe, Zn, Pb, As, Cd and Hg in a biological fluid.

4. A method for producing an immunoenzymatic electroanalytical sensor or an electroanalytical sensor for the detection of at least a metal selected from the group consisting of Cu, Fe, Zn, Pb, As, Cd and Hg in a biological fluid comprising the steps of:
- providing a cellulose support;
- delimiting on the cellulose support a hydrophilic working area by depositing a hydrophobic material;
- depositing on the hydrophilic working area of the cellulose support, a single aqueous solution of the metal precursor not including an additional reducing agent, in the form of acid or salt, in a concentration from 1 to 6 mM;
- drying the cellulose support at a temperature from 65°C to 80°C for a time from 10 to 40 minutes so that metal nanoparticles are formed on the cellulose support;
- printing, on the hydrophilic working area of the cellulose support with metal nanoparticles, at least one working electrode, one reference electrode and a counter-electrode by screen-printing by depositing conductive inks in a sequence.

5. The method according to claim 4,
wherein the aqueous solution is a HAuCl₄ solution.

6. An immunoenzymatic electroanalytical sensor for detecting at least a metal selected from the group consisting of Cu, Fe, Zn, Pb, As, Cd and Hg in a biological fluid, comprising a cellulose support functionalised by metal nanoparticles formed in situ by the method of claim 1, on which a hydrophobic area delimits a hydrophilic working area, said hydrophilic working area comprising at least one working electrode, one reference electrode and one counter-electrode printed by screen-printing.

7. The sensor according to claim 6, wherein the metal nanoparticles are made of gold, the working electrode is made of gold, the reference electrode is made of silver/silver chloride and the counter-electrode is made of graphite.

8. The sensor according to claim 6, wherein the cellulose support is filter paper, the metal nanoparticles are made of gold, the marker is copper, the working electrode is made of gold, the reference electrode is made of silver/silver chloride and the counter-electrode is made of graphite.

9. A method for detecting at least a metal selected from the group consisting of Cu, Fe, Zn, Pb, As, Cd and Hg or a marker detectable by means of an immunoenzymatic sensor in a biological fluid comprising the steps of:
- providing a sensor according to any of claims 6 to 8;
- adding on said sensor an amount of biological fluid from 1 to 80 µl, preferably 20 to 40 µl, optionally subjected to a concentration or dilution pre-treatment;
- applying a potential difference between the electrodes of the sensor;
- detecting a current signal by means of a potentiostat, the signal being proportional to the amount of the at least one metal or marker in the biological fluid.

## Patentansprüche

1. Verfahren zur Funktionalisierung eines Celluloseträgers in situ mit Metallnanopartikeln mit elektrokatalytischen Eigenschaften, wobei das Verfahren die folgenden Schritte umfasst:
- Aufbringen einer einzigen wässrigen Lösung des Metallvorläufers, die kein zusätzliches Reduktionsmittel in Form einer Säure oder eines Salzes enthält, in einer Konzentration von 1 bis 6 mM auf einen Celluloseträger; und
- Trocknen des Celluloseträgers bei einer Temperatur von 65°C bis 80°C für einen Zeitraum von 10 bis 40 Minuten.

2. Celluloseträger, funktionalisiert mit in situ gebildeten Metallnanopartikeln, erhalten durch das Verfahren nach Anspruch 1.

3. Verwendung des Celluloseträgers nach Anspruch 2 bei der Herstellung von immunoenzymatischen elektroanalytischen Sensoren oder elektroanalytischen Sensoren zum Nachweis zumindest eines Metalls, ausgewählt aus der Gruppe bestehend aus Cu, Fe, Zn, Pb, As, Cd und Hg, in einem biologischen Fluid.

4. Verfahren zur Herstellung eines immunoenzymatischen elektroanalytischen Sensors oder eines elektroanalytischen Sensors zum Nachweis zumindest eines Metalls, ausgewählt aus der Gruppe bestehend aus Cu, Fe, Zn, Pb, As, Cd und Hg, in einem biologischen Fluid, umfassend die folgenden Schritte:
- Bereitstellen eines Celluloseträgers;
- Abgrenzen eines hydrophilen Arbeitsbereichs auf dem Celluloseträger durch Aufbringen eines hydrophoben Materials;
- Aufbringen einer einzigen wässrigen Lösung des Metallvorläufers, die kein zusätzliches Reduktionsmittel enthält, in Form einer Säure oder eines Salzes in einer Konzentration von 1 bis 6 mM auf den hydrophilen Arbeitsbereich des Celluloseträgers;
- Trocknen des Celluloseträgers bei einer Temperatur von 65°C bis 80°C für einen Zeitraum von 10 bis 40 Minuten, so dass Metallnanopartikel auf dem Celluloseträger gebildet werden;
- Aufdrucken zumindest einer Arbeitselektrode, einer Referenzelektrode und einer Gegenelektrode auf den hydrophilen Arbeitsbereich des Celluloseträgers mit Metallnanopartikeln durch Siebdruck, indem leitfähige Tinten nacheinander aufgebracht werden.

5. Verfahren nach Anspruch 4, wobei die wässrige Lösung eine HAuCl₄-Lösung ist.

6. Immunoenzymatischer elektroanalytischer Sensor zum Nachweis zumindest eines Metalls, ausgewählt aus der Gruppe bestehend aus Cu, Fe, Zn, Pb, As, Cd und Hg, in einem biologischen Fluid, umfassend einen Celluloseträger, der durch Metallnanopartikel funktionalisiert ist, die in situ durch das Verfahren nach Anspruch 1 gebildet wurden, auf dem ein hydrophober Bereich einen hydrophilen Arbeitsbereich abgrenzt, wobei der hydrophile Arbeitsbereich zumindest eine Arbeitselektrode, eine Referenzelektrode und eine Gegenelektrode umfasst, die durch Siebdruck aufgedruckt wurden.

7. Sensor nach Anspruch 6, wobei die Metallnanopartikel aus Gold bestehen, die Arbeitselektrode aus Gold besteht, die Referenzelektrode aus Silber/Silberchlorid besteht und die Gegenelektrode aus Graphit besteht.

8. Sensor nach Anspruch 6, wobei der Celluloseträger Filterpapier ist, die Metallnanopartikel aus Gold bestehen, der Marker Kupfer ist, die Arbeitselektrode aus Gold besteht, die Referenzelektrode aus Silber/Silberchlorid besteht und die Gegenelektrode aus Graphit besteht.

9. Verfahren zum Nachweis zumindest eines Metalls, ausgewählt aus der Gruppe bestehend aus Cu, Fe, Zn, Pb, As, Cd und Hg, oder eines Markers, nachweisbar mithilfe eines immunoenzymatischen Sensors, in einem biologischen Fluid, umfassend die folgenden Schritte:
- Bereitstellen eines Sensors nach einem der Ansprüche 6 bis 8;
- Zugeben einer Menge eines biologischen Fluids von 1 bis 80 µl, bevorzugt 20 bis 40 µl, das wahlweise einer Konzentrations- oder Verdünnungsvorbehandlung unterzogen wurde, auf den Sensor;
- Anlegen einer Potentialdifferenz zwischen den Elektroden des Sensors;
- Erfassen eines Stromsignals mithilfe eines Potentiostaten, wobei das Signal proportional zu der Menge des zumindest einen Metalls oder Markers in dem biologischen Fluid ist.

## Revendications

1. Procédé de fonctionnalisation in situ d'un support cellulosique avec des nanoparticules métalliques ayant des propriétés électrocatalytiques, le procédé comprenant les étapes de :
- dépôt sur un support cellulosique d'une solution aqueuse unique du précurseur métallique ne comprenant pas d'agent réducteur supplémentaire, sous forme d'acide ou de sel, à une concentration de 1 à 6 mM ; et
- séchage du support cellulosique à une température de 65 °C à 80 °C pendant une durée de 10 à 40 minutes.

2. Support cellulosique fonctionnalisé par des nanoparticules métalliques formées in situ obtenu par le procédé selon la revendication 1.

3. Utilisation du support cellulosique selon la revendication 2 dans la fabrication de capteurs électroanalytiques immunoenzymatiques ou de capteurs électroanalytiques pour la détection d'au moins un métal choisi dans le groupe constitué par Cu, Fe, Zn, Pb, As, Cd et Hg dans un fluide biologique.

4. Procédé de production d'un capteur électroanalytique immunoenzymatique ou d'un capteur électroanalytique pour la détection d'au moins un métal choisi dans le groupe constitué par Cu, Fe, Zn, Pb, As, Cd et Hg dans un fluide biologique comprenant les étapes de :
- fourniture d'un support cellulosique ;
- délimitation sur le support cellulosique d'une zone de travail hydrophile par dépôt d'un matériau hydrophobe ;
- dépôt sur la zone de travail hydrophile du support cellulosique, d'une solution aqueuse unique du précurseur métallique ne comprenant pas d'agent réducteur supplémentaire, sous forme d'acide ou de sel, à une concentration de 1 à 6 mM ;
- séchage du support cellulosique à une température de 65 °C à 80 °C pendant une durée de 10 à 40 minutes de sorte que des nanoparticules métalliques soient formées sur le support cellulosique ;
- impression, sur la zone de travail hydrophile du support cellulosique avec des nanoparticules métalliques, d'au moins une électrode de travail, une électrode de référence et une contre-électrode par sérigraphie par dépôt d'encres conductrices en séquence.

5. Procédé selon la revendication 4, dans lequel la solution aqueuse est une solution de HAuCl4.

6. Capteur électroanalytique immunoenzymatique pour la détection d'au moins un métal choisi dans le groupe constitué par Cu, Fe, Zn, Pb, As, Cd et Hg dans un fluide biologique, comprenant un support cellulosique fonctionnalisé par des nanoparticules métalliques formées in situ par le procédé de la revendication 1, sur lequel une zone hydrophobe délimite une zone de travail hydrophile, ladite zone de travail hydrophile comprenant au moins une électrode de travail, une électrode de référence et une contre-électrode imprimées par sérigraphie.

7. Capteur selon la revendication 6, dans lequel les nanoparticules métalliques sont constituées d'or, l'électrode de travail est constituée d'or, l'électrode de référence est constituée d'argent/chlorure d'argent et la contre-électrode constituée de graphite.

8. Capteur selon la revendication 6, dans lequel le support cellulosique est du papier filtre, les nanoparticules métalliques sont constituées d'or, le marqueur est en cuivre, l'électrode de travail est constituée d'or, l'électrode de référence est constituée d'argent/chlorure d'argent et la contre-électrode est constituée de graphite.

9. Procédé de détection d'au moins un métal choisi dans le groupe constitué par Cu, Fe, Zn, Pb, As, Cd et Hg ou un marqueur détectable au moyen d'un capteur immunoenzymatique dans un fluide biologique comprenant les étapes de :
- fourniture un capteur selon l'une quelconque des revendications 6 à 8 ;
- ajout sur ledit capteur une quantité de fluide biologique de 1 à 80 µl, de préférence de 20 à 40 µl, éventuellement soumis à un prétraitement par concentration ou dilution ;
- application d'une différence de potentiel entre les électrodes du capteur ;
- détection d'un signal de courant au moyen d'un potentiostat, le signal étant proportionnel à la quantité d'au moins un métal ou marqueur dans le fluide biologique.
